# EUROPÄISCHE PATENTSCHRIFT

(11) **EP 2 584 030 B1**
(45) Veröffentlichungstag und Bekanntmachung des Hinweises auf die Patenterteilung: **10.09.2014**
(21) Anmeldenummer: 12007222.8
(22) Anmeldetag: 18.10.2012
(51) Int. Cl.: C12M 1/00

(54) **Verfahren und Vorrichtung zur Kultivierung von phototrophen Organismen**
Method and apparatus for cultivating phototropic organisms
Procédé et dispositif destinés à cultiver des organismes phototrophes

(30) Priorität: 18.10.2011 DE 102011116237
(43) Veröffentlichungstag der Anmeldung: 24.04.2013
(73) Patentinhaber: Warscheid, Thomas, 26215 Wiefelstede (DE)
(72) Erfinder: Warscheid, Thomas, 26215 Wiefelstede (DE)
(74) Vertreter: Jabbusch, Matthias

(56) Entgegenhaltungen:
- EP-A1- 2 228 432
- WO-A1-96/23865
- WO-A1-2008/145719
- WO-A1-2011/031161
- WO-A2-2010/042484
- WO-A2-2010/102316
- DE-A1-102009 051 588
- GB-A- 2 460 114
- GB-A- 2 473 865
- US-A- 2 732 663

## Beschreibung

Die Erfindung betrifft ein Verfahren zur Kultivierung von phototrophen Organismen in wenigstens einem Bioreaktor, bei dem ein wässriges Substrat mindestens einen die phototrophen Organismen aufnehmenden Reaktorraum durchströmt, wobei Nährstoffe aus dem Substrat von den Organismen aufgenommen werden.

Von den phototrophen Organismen, insbesondere Algen und Cyanobakterien, wird CO₂ unter Lichteinwirkung zu Biomasse verstoffwechselt. Dabei wird bei den bekannten Verfahren häufig entweder die Biomasse selbst oder ein von den phototrophen Organismen erzeugtes Stoffwechselprodukt gewonnen. Die Kultivierung der phototrophen Organismen erfolgt zumeist in einem wässrigen Substrat oder Nährmedium, das die für ein Wachstum der Organismen benötigten Nährstoffe bereitstellt. Als Nährstoffe sind hier insbesondere Mineralstoffe und Spurenelemente sowie eine Stickstoffquelle anzuführen. Weiterhin wird von den phototrophen Organismen wenigstens eine CO₂-Quelle benötigt. Für diese CO₂-Quelle werden bevorzugt Rauch- beziehungsweise Abgase, beispielsweise aus Kraftwerken, eingesetzt, um die darin enthaltene CO₂-Konzentration zu reduzieren. Die exakte Zusammenstellung des Substrats beziehungsweise Nährmediums ist neben CO₂ und Licht somit entscheidend für die Kultivierung phototropher Organismen, so dass auch für das Substrat beziehungsweise Nährmedium, wie bei der CO₂-Quelle nach kostengünstigen Alternativen gesucht wird. Dabei soll eine gleichbleibende Zusammensetzung der Nährstoffe erreicht werden.

Der Erfindung liegt daher die Aufgabe zugrunde, ein Verfahren bereitzustellen, dass eine gleichbleibende Zusammensetzung des Substrats gewährleistet und kostengünstig betrieben werden kann.

Diese Aufgabe ist verfahrensmäßig durch die Merkmale des Patentanspruchs 1 gelöst. Vorrichtungsmäßig wird die Aufgabe mit den Merkmalen des Patentanspruchs 7 gelöst. Weiterbildungen und vorteilhafte Ausgestaltungen ergeben sich aus den Unteransprüchen.

Das erfindungsgemäße Verfahren zeichnet sich dadurch aus, dass das Substrat über wenigstens einen mineralischen Festkörper geführt wird und durch den das Substrat hindurchströmt, wobei das Substrat an dem wenigstens einen mineralischen Festkörper mit zumindest einem Teil der Nährstoffe angereichert wird. Dabei werden einzelne Partikel von dem wenigstens einen mineralischen Festkörper abgelöst und in dem Substrat mitgeführt. Über die dem mineralischen Festkörper zugeführte Menge an Substrat sowie die Fließgeschwindigkeit des Substrats ist somit auf besonders einfache Weise eine Regelung des Nährstoff- beziehungsweise Partikelübergangs von dem mineralischen Festkörper in das Substrat ermöglicht. Bei konstanter Durchflussmenge des Substrats ist auch eine gleichbleibende Nährstoffanreicherung gewährleistet. Das durch den Reaktorraum strömende Substrat erzeugt in dem Reaktorraum zudem eine gute Durchmischung. Der mineralische Festkörper kann beispielsweise aus einer Schüttung eines Granulats, einem porösen Gestein oder einer zu überströmenden Fläche bestehen. Da immer wieder Partikel von dem mineralischen Festkörper abgelöst werden, muss dieser in regelmäßigen Abständen erneuert beziehungsweise ergänzt werden, um eine gleichbleibende Zusammensetzung des Substrats zu gewährleisten. Weitere Nährstoffe, wie Stickstoff, sind durch eine geeignete Auswahl eines anzureichernden Ausgangssubstrats zuzuführen, wobei die benötigten Nährstoffe insbesondere in häuslichen Abwässern enthalten sind.

In dem Reaktorraum werden die Nährstoffe des angereicherten Substrats von den phototrophen Organismen aufgenommen. Damit die phototrophen Organismen in dem Reaktorraum Biomasse bilden, wird zudem noch eine CO₂,-Quelle beispielsweise ein Abgas beziehungsweise ein Rauchgas zugeführt. Mit zunehmendem Wachstum der phototrophen Organismen nimmt die Biomassekonzentration in dem Reaktorraum zu, wobei mit zunehmender Biomassekonzentration mehr Organismen mit dem Substrat aus dem Reaktorraum ausgeschwemmt werden. Die aus dem Reaktorraum ausgeschwemmten Organismen werden dann bevorzugt von dem Substrat abgetrennt und können beispielsweise energetisch genutzt werden. Das Abtrennen der Organismen beziehungsweise deren Biomasse von dem Substrat erfolgt beispielsweise durch ein Absetzen dieser und Abschöpfen des Substrats oder durch Filtern des Substrats, wobei die Organismen beziehungsweise die Biomasse in einem Filter aufgefangen werden.

Als phototrophe Organismen werden vor allem in der Algenbiotechnologie bekannte Grünalgen, wie Chlorella vulgaris, Chlamydomonas reinhardtii, Haematococcus pluvialis, Botryococcus braunii und Phaeodactylum spec, sowie Cyanobakterien, wie Synechoccoccus spec., eingesetzt. Weiterhin sind Wildstämme und Mischkulturen von Algen und Cyanobakterien aus subarktischen, gemäßigten und tropischen Zonen vorgesehen.

Um die bei dem Verfahren anfallende Abwassermenge zu reduzieren, ist vorgesehen, dass das Substrat in einem Kreislauf geführt wird, bei dem das Substrat nach dem Abtrennen der ausgeschwemmten Organismen erneut in den Reaktorraum eingeleitet wird. Die dem Substratkreislauf neu zuzuführende Menge an beispielsweise häuslichem Abwasser ist somit möglichst gering. Das in den Reaktorraum rückgeführte Substrat wird an dem wenigstens einen mineralischen Festkörper immer wieder mit Nährstoffen angereichert, die dann an die phototrophen Organismen abgegeben werden.

Vorteilhafterweise wird das Substrat mit natürlichen Mineral und/oder Nährstoffen angereichert, bevor es in den Reaktorraum eingeleitet wird. Die Anreicherung mit Nährstoffen ist dann räumlich vom Reaktorraum mit den phototrophen Organismen getrennt, wodurch insbesondere die Nährstoffanreicherung auf einfachere Weise überwacht und geregelt werden kann.

Um die Kultivierung weitestgehend unabhängig von äußeren Temperaturen durchführen zu können, ist vorgesehen, dass zumindest der Reaktorraum temperiert wird. Ein temperierter Bioreaktor kann unter anderem auch an Hausfassaden oder dergleichen montiert werden. Das Temperieren umfasst einerseits ein Beheizen des Reaktorraums bei niedrigen Außentemperaturen, beispielsweise im Winter, und ein Kühlen des Reaktorraumes bei hohen Temperaturen, beispielsweise im Sommer. Je nach Standort ist es auch möglich, den Reaktorraum nur zu Beheizen oder nur zu Kühlen.

Zum Beheizen des Reaktorraumes eignet sich insbesondere Rauchgas, wobei das Rauchgas zumindest teilweise in den Reaktorraum eingeleitet wird. In dem Reaktorraum dient das Rauchgas als CO₂-Quelle für die phototrophen Organismen und verstärkt gleichzeitig die Durchmischung des Substrats im Reaktorraum. Eine besonders effektive Durchmischung ist erreicht, wenn das Rauchgas und das Substrat im Gegenstrom zueinander geführt werden.

Weiterhin betrifft die Erfindung eine Vorrichtung zur Kultivierung von phototrophen Organismen, mit zumindest einem Bioreaktor, der wenigstens einen die phototrophen Organismen aufnehmenden Reaktorraum mit einem zumindest abschnittsweise lichtdurchlässigen Gehäuse aufweist. Diese Vorrichtung zeichnet sich dadurch aus, dass der Bioreaktor einen durch den Reaktorraum führenden Strömungskanal für ein Substrat zur Nährstoffversorgung der Organismen aufweist, wobei in wenigstens einem Abschnitt des Strömungskanals zumindest ein mineralischer Festkörper angeordnet ist. In dem Strömungskanal fließendes Substrat wird über den mineralischen Festkörper geführt und an dem mineralischen Festkörper mit wenigstens einem Teil der von den phototrophen Organismen benötigten Nährstoffen, insbesondere mineralischen Nährstoffen, angereichert. Der mineralische Festkörper kann beispielsweise als in einem Abschnitt des Strömungskanals angeordnetes Granulat oder einzelner poröser Festkörper ausgebildet sein, durch den das Substrat hindurchströmt. Als mineralischer Festkörper sind unter anderem Basalt, Bimsstein und/oder Quarz geeignet.

Dem Reaktorraum ist vorteilhafterweise wenigstens eine Entnahmeeinrichtung für aus dem Reaktorraum ausgeschwemmte Organismen zugeordnet. Mit dem Substrat werden auch Organismen aus dem Reaktorraum ausgetragen, die in der Entnahmeeinrichtung aus dem Substrat abgeschöpft werden. Die Entnahmeeinrichtung weist dazu in einer bevorzugten Ausgestaltung ein Absetzbecken auf, in dem sich die mit den phototrophen Organismen im Reaktorraum produzierte Biomasse absetzt. Das Substrat wird dabei über einen Substratüberlauf aus dem Absetzbecken abgeführt. Die im Absetzbecken zurückbleibende Biomasse kann unter anderem energetisch genutzt werden.

Um ein möglichst geschlossenes System zu erhalten, ist vorgesehen, dass zwischen dem Reaktorraum und der Entnahmeeinrichtung ein Substratkreislauf ausgebildet ist. Dem Absetzbecken der Entnahmeeinrichtung ist dann ein Pumpbecken zuzuordnen, in welches das aufgereinigte Substrat über den Substratüberlauf aus dem Absetzbecken geleitet wird. Mittels einer bevorzugt solarbetriebenen Substratpumpe wird das aufgereinigte Substrat dann aus dem Pumpbecken in den Reaktorraum zurückgefördert. In dem Reaktorraum selbst strömt das Substrat auf einfache Weise schwerkraftbedingt wieder zu der Entnahmeeinrichtung. Das in dem Strömungskanal des Bioreaktors fließende Substrat wird somit immer wieder über den wenigstens einen mineralischen Festkörper geführt und mit Nährstoffen angereichert, so dass nur wenig Ausgangssubstrat, zum Beispiel häusliches Abwasser, neu hinzugegeben werden muss.

Eine wesentlich verbesserte Ausnutzung des Reaktorraums ist dadurch erreichbar, dass in einem vom Reaktorraum begrenzten Bereich des Strömungskanals wenigstens abschnittsweise zumindest eine Schüttung aus Granulat angeordnet ist. Der Reaktorraum weist dadurch eine wesentlich vergrößerte Oberfläche auf, so dass mit dem hier entwickelten Bioreaktor möglichst natürliche und optimale Aufwuchsbedingungen für terestische und limnische Algen, die häufig Materialoberflächen gegenüber einer künstlichen Nährlösung beziehungsweise einem Substrat bevorzugen, geschaffen sind. Das Granulat besteht bevorzugt aus Quarz, insbesondere eisenhaltigem Quarzgranulat, mit einem Durchmesser von ca. 3 bis 12 mm. Auf diesem Quarzgranulat bilden die phototrophen Organismen Biofilme aus, wobei das lichtdurchlässige Quarzgranulat gleichzeitig auch einen Schutz gegen zu intensive Sonneneinstrahlung bietet. Die phototrophen Organismen ziehen sich bei zu intensiver Sonneneinstrahlung auf weniger stark bestrahlte Bereiche zurück. Gleichzeitig gewährleistet Quarz eine optimale Eisenversorgung der phototrophen Organismen.

Für eine optimale Nährstoffversorgung der phototrophen Organismen, ist weiter vorgesehen, dass in dem Strömungskanal wenigstens eine dem Reaktorraum in Strömungsrichtung des Substrats vorgeschaltete Nährstoffanreichungseinrichtung mit wenigstens einem mineralischen Festkörper angeordnet ist. Nährstoffanreicherung und Reaktorraum mit phototrophen Organismen sind somit vorteilhaft räumlich voneinander getrennt. Zudem können die mineralischen Festkörper in der Nährstoffanreichungseinrichtung auf einfache Weise ausgetauscht werden. Hierzu kann beispielsweise eine Art Kartusche in den Strömungskanal des Substrats eingesetzt werden. Die Nährstoffanreichungseinrichtung kann dabei insbesondere als ein Überlaufbehälter ausgestaltet sein, so dass eine möglichst lange Verweildauer des Substrats in der Nährstoffanreicherungseinrichtung gewährleistet ist.

Das Gehäuse des Reaktorraums weist zumeist eine lichtdurchlässige Vorderwand und eine parallel zu der Vorderwand angeordnete Rückwand auf, die zusammen mit Seitenwänden den quader- beziehungsweise plattenförmigen Reaktorraum umschließen. Innerhalb des Reaktorraums sind vorteilhafterweise Stege angeordnet, die einen mäanderartigen Strömungskanal von einem oberen Ende des Reaktorraumes zu einem unteren Ende des Reaktorraumes vorgeben. Die Stege sind abwechselnd an zueinander gegenüberliegenden Seitenwänden angesetzt und liegen mit je einem Rand sowohl an der Vorderwand als auch an der Rückwand des Gehäuses an. Dabei gewährleisten die Stege, dass der Reaktorraum vollständig von Substrat durchströmt wird und Toträume weitestgehend vermieden sind. Bevorzugtes Material für zumindest die Vorderwand und die Stege ist zum Beispiel lichtbeständiges und lichtdurchlässiges Polycarbonat, wobei Vorderwand und Stege, insbesondere auch UV-geschützt ausgebildet sind. Für das Gehäuse können auch entsprechend modifizierte Doppelstegplatten oder dergleichen verwendet werden.

Zudem ist vorgesehen, dass dem Reaktorraum eine Temperiereinheit zugeordnet ist. Für die im Reaktorraum zu kultivierenden phototrophen Organismen ist somit immer eine das Wachstum fördernde Temperatur einstellbar. Dazu schließt die Temperiereinheit vorteilhafterweise flächig an die Rückwand des Reaktorraums an, so dass über die gesamte Rückwand des Reaktorraums eine Beheizung erfolgt. Die Beheizung kann vorteilhafterweise mit zum Beispiel Rauchgas beziehungsweise Heizungsabluft erfolgen.

In den Bioreaktor als CO₂-Quelle eingespeistes Rauchgas kann somit gleichzeitig für die Beheizung des Reaktorraumes genutzt werden, indem das zugeführte Rauchgas in den Reaktorraum beziehungsweise in die Temperiereinheit verteilt wird. Dazu ist dem Reaktorraum vorteilhafterweise wenigstens ein Begasungsventil zugeordnet, das zwischen dem Reaktorraum und der Temperiereinheit angeordnet ist. Dabei wird bevorzugt erst die Temperiereinheit vollständig von dem Rauchgas durchströmt, bevor dieses zu dem Begasungsventil geleitet wird, um eine Abkühlung des meist zu heißen Rauchgases zu erreichen. Eine besonders gute Durchmischung des Substrats im Reaktorraum wird dadurch erreicht, dass dem Reaktorraum wenigstens ein Begasungsschlauch zugeordnet ist, der zumindest teilweise in dem Reaktorraum angeordnet ist und in dem Reaktorraum zumindest abschnittsweise porös ausgebildet ist. Der Begasungsschlauch gewährleistet eine besonders gleichmäßige Begasung des Reaktorraumes mit CO₂ über die porösen Abschnitte, wobei der Begasungsschlauch bevorzugt im unteren Drittel, insbesondere im unteren Viertel des Reaktorraumes angeordnet ist. Eine weitere Möglichkeit eine großflächige Begasung mit Rauchgas zu schaffen, besteht darin, dass zwischen dem Reaktorraum und der Temperiereinheit Glasfritten angeordnet sind, wobei die Glasfritten für in der Temperiereinheit befindliches Rauchgas zum Reaktorraum hin durchlässig ausgebildet sind. Dazu ist in einer bevorzugten Ausgestaltung zwischen jedem zweiten Steg jeweils wenigstens eine Glasfritte in der Rückwand angeordnet.

Schließlich zeichnet sich die Vorrichtung dadurch aus, dass diese als Fassadenelement oder Dachelement ausgebildet ist. Fassaden und/oder Dächer können so auf einfache Weise zur Energiegewinnung genutzt werden. Die für das Wachstum der phototrophen Organismen benötigten großen Flächen, können dabei auch als gestalterisches Element verwendet werden. Hierfür bieten sich insbesondere bisher ungenutzte, großflächige Fassaden von Industriebauten an, da die benötigten Rauchgase zumeist in großen Mengen vor Ort anfallen. Neben Industriebauten liegt ein weiterer Anwendungsschwerpunkt in Kleinanlagen für Wohnhäuser, um zum Beispiel eine bestimmte Menge der im Haus benötigten Energie bereitzustellen. Die Entnahmeeinrichtung kann dabei mit einer Heizungsanlage kombiniert werden, in der die entnommene Biomasse verfeuert wird. Der Reaktorraum und die Entnahmeeinrichtung wären dann räumlich voneinander getrennt einzurichten und miteinander zu verbinden. Weiterhin sind auch Anwendungen im Innenbereich beispielsweise als Wandelement oder Raumteiler möglich, wodurch das Raumklima verbessert werden kann.

Ein Ausführungsbeispiel der Erfindung, aus dem sich weitere erfinderische Merkmale ergeben, ist in der Zeichnung dargestellt. Es zeigen:
- Fig. 1:: eine schematische Darstellung der Vorrichtung; und
- Fig. 2:: eine Explosionsdarstellung von Reaktorraum und Temperiereinheit in perspektivische Ansicht.
- Fig. 3:: eine zweite Ausführungsvariante des Reaktorraumes mit der Temperiereinheit in perspektivische Ansicht.

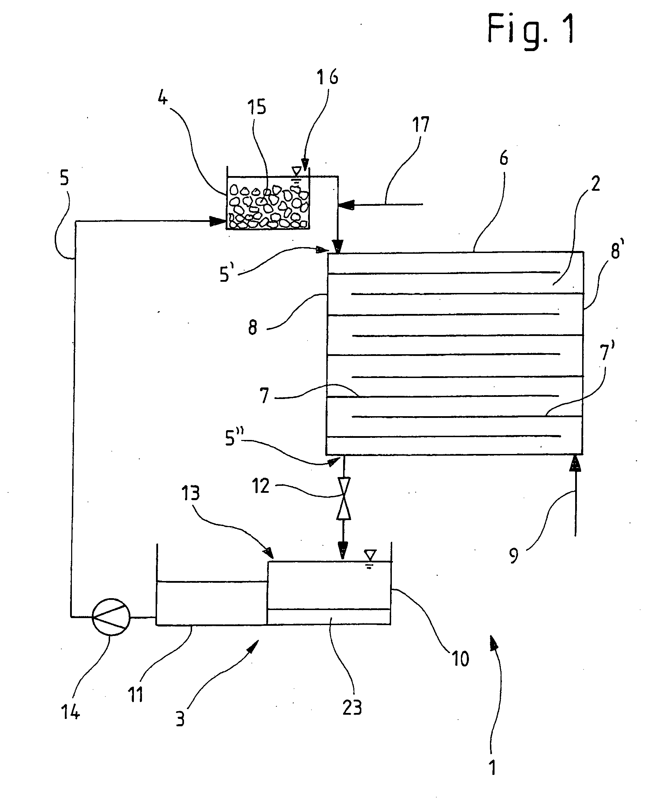

Die Fig. 1 zeigt einen Bioreaktor 1 mit einem Reaktorraum 2, einer Entnahmeeinrichtung 3 sowie einer Nährstoffanreichungseinrichtung 4, die durch einen Strömungskanal 5 miteinander verbunden sind. Der Strömungskanal 5 ist als Substratkreislauf ausgebildet und weist eine für ein in dem Strömungskanal 5 geführtes Substrat vorgegebene Strömungsrichtung auf. Diese Strömungsrichtung des Strömungskanals 5 ist in Fig. 1 mit Pfeilspitzen gekennzeichnet, wobei die Nährstoffanreichungseinrichtung 4 dem Reaktorraum 2 vorgeschaltet ist und die Entnahmeeinrichtung 3 dem Reaktorraum 2 nachgeschaltet ist. Der Reaktorraum 2 ist von einem Gehäuse 6 umgeben, wobei der Strömungskanal 5 an einem oberen Anschluss 5' in den Reaktorraum 2 hineingeführt ist und an einem unteren Anschluss 5" aus dem Reaktorraum 2 hinausgeführt ist. In dem Reaktorraum 2 sind Stege 7, 7' angeordnet, die abwechselnd an einander gegenüberliegenden Seitenwänden 8, 8' des Gehäuses 6 angesetzt sind und eine mäanderartige Führung des Strömungskanals 5 in dem Reaktorraum 2 vorgeben. Dem Gehäuse 6 ist weiterhin eine CO₂-Leitung 9 zugeordnet, die in den Reaktorraum 2 einmündet.

Die Entnahmeeinrichtung 3 weist ein Absetzbecken 10 und ein Pumpbecken 11 auf, wobei das Absetzbecken 10 dem Pumpbecken 11 in Strömungsrichtung des Substrats vorgeschaltet ist. Der Zulauf von Substrat aus dem Reaktorraum 2 in das Absetzbecken 9 wird über ein in dem Strömungskanal 5 zwischen dem Reaktorraum 2 und dem Absetzbecken 9 angeordnetes Absperrventil 12 geregelt. Das Absetzbecken 10 und das Pumpbecken 11 sind über einen ersten Substratüberlauf 13 vom Absetzbecken 10 in das Pumpbecken 11 miteinander verbunden.

Zwischen der Entnahmeeinrichtung 3 und der Nährstoffanreichungseinrichtung 4 ist in dem Substratkreislauf des Strömungskanals 5 eine Substratpumpe 14 angeordnet, mit der das Substrat zu der Nährstoffanreichungseinrichtung 4 förderbar ist. Die Nährstoffanreichungseinrichtung 4 besteht aus einem Überlaufbehälter, der mit mineralischen Festkörpern 15 gefüllt ist und einen zweiten Substratüberlauf 16 aufweist. Zwischen der Nährstoffanreichungseinrichtung 4 und dem Reaktorraum 2 mündet eine Abwasserleitung 17 in den Strömungskanal 5.

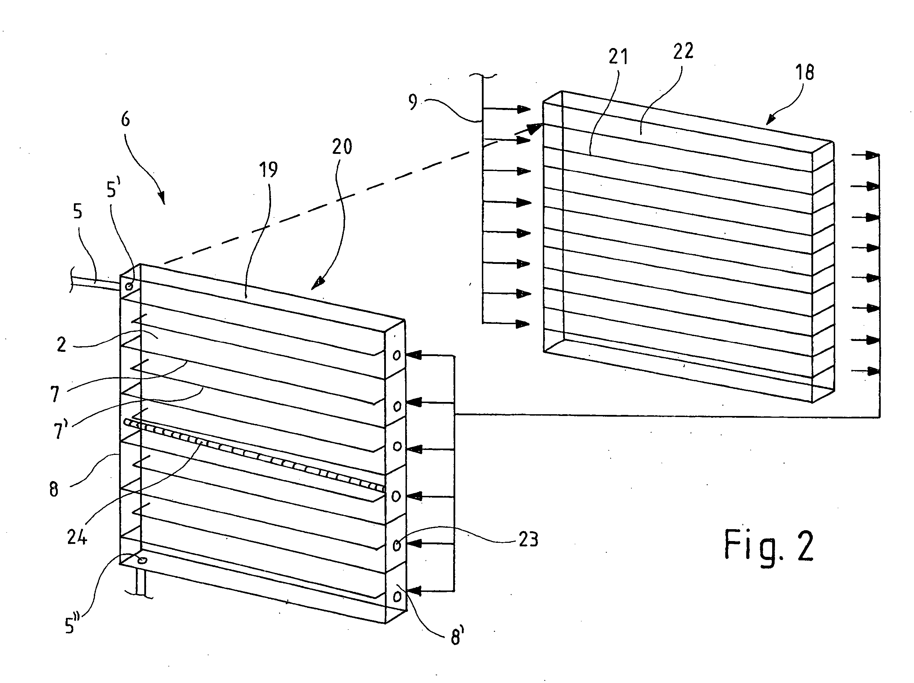

In Fig. 2 ist das Gehäuse 6 des Reaktorraums 2 mit den Anschlüssen 5', 5" des Strömungskanals 5 sowie einer Temperiereinheit 18 dargestellt. Der Reaktorraum 2 ist hauptsächlich von den Seitenwänden 8, 8', von einer lichtdurchlässigen Vorderwand 19 und von einer Rückwand 20, an die die Temperiereinheit 18 ansetzbar ist, begrenzt. Um die Anschlüsse der Temperiereinheit 18 sowie die CO₂-Leitung 9 mit ihren Verzweigungen und Verbindungen zum Reaktorraum 2 in Fig. 2 hervorheben zu können, sind der Reaktorraum 2 und die Temperiereinheit 18 mit Abstand zueinander dargestellt. Dabei entspricht die Temperiereinheit 18 in ihren Abmessungen weitestgehend denen des Reaktorraums 2 und ist bevorzugt mit in das Gehäuse 6 integriert. Die Temperiereinheit 18 weist ebenso wie der Reaktorraum 2 Stege 21 auf, die die Temperiereinheit 18 in parallel zueinander angeordnete Strömungskanäle 22 unterteilen. In Strömungsrichtung vor den Strömungskanälen 22 verzweigt sich die CO₂-Leitung 9 und wird in den Strömungskanälen 22 über die Temperiereinheit 18 in den Reaktorraum 2 geführt. Am Reaktorraum 2 weist die CO₂-Leitung 9 zwischen den Stegen 7, 7' des Reaktorraums 2 angeordnete, in den Reaktorraum 2 gerichtete Einspritzdüsen 23 auf, wobei auch ein oder mehrere poröse Schläuche 24 in dem Reaktorraum 2 angeordnet sein können, so dass das bevorzugt zum Temperieren verwendete Rauchgas gleichzeitig eine gute CO₂-Versorgung der phototrophen Organismen gewährleistet.

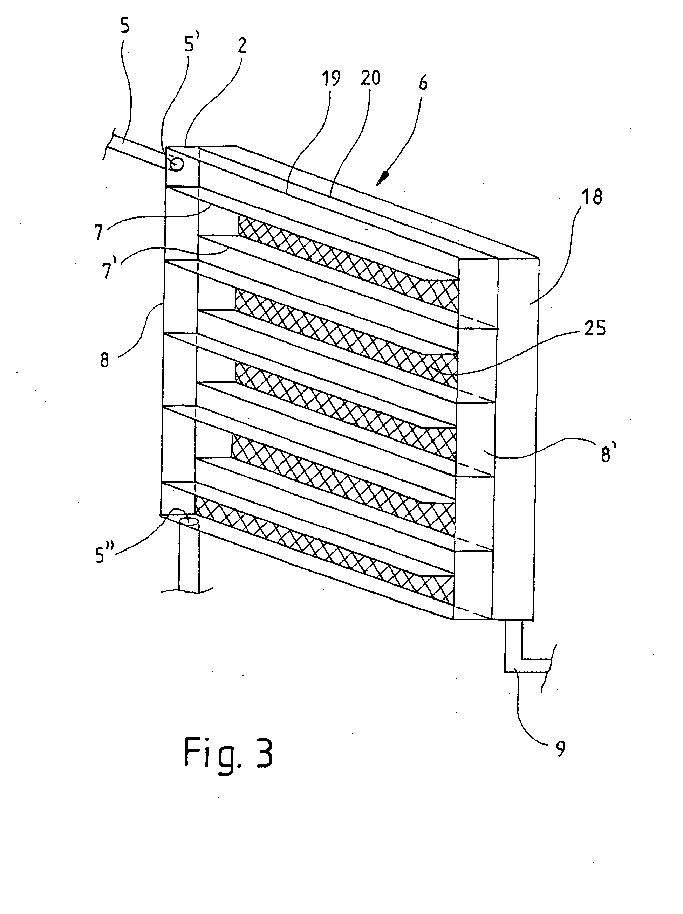

Eine alternative Anordnung der Temperiereinheit 18 ist in Fig. 3 dargestellt. Dabei sind zwischen der Temperiereinheit 18 und dem Reaktorraum 2 in der Rückwand 20 Glasfritten 25 angeordnet. Die Glasfritten 25 sind mit Abstand zueinander vorgesehen, so dass zwischen jedem zweiten Steg 7,7' jeweils ein Bereich mit einer Glasfritte 25 ausgebildet ist.

Im Betrieb ist der Reaktorraum 2 mit Quarzgranulat gefüllt, auf dessen Oberfläche phototrophe Organismen einen Biofilm ausbilden. Das Substrat wird in die Nährstoffanreicherungseinrichtung 4 gefördert und nimmt in der Nährstoffanreicherungseinrichtung 4 mineralische Partikel der mineralischen Festkörper 15 auf. Anschließend wird das Substrat mit nährstoffreichem, insbesondere stickstoffhaltigem Abwasser aus der Abwasserleitung 17 versetzt und gelangt in den Reaktorraum 2. In dem Reaktorraum 2 werden die im Substrat enthaltenen Nährstoffe an die phototrophen Organismen abgegeben. Gleichzeitig wird mit dem durch den Reaktorraum 2 strömenden Substrat sowie dem im Gegenstrom zum Substrat eingeleiteten CO₂ eine optimale Durchmischung im Reaktorraum 2 erreicht, wodurch überschüssige Biomasse 26 der phototrophen Organismen von dem Quarzgranulat abgeschert und mit dem Substrat aus dem Reaktorraum 2 ausgetragen wird. Das ausgetragene Substrat mit der Biomasse 26 gelangt in das Absetzbecken 10, in dem sich die Biomasse 26 am Boden absetzt. Das Substrat hingegen wird mittels Substratüberlauf 13 in das Pumpbecken 11 überführt, aus dem es dann mit der Substratpumpe 14 zur Nährstoffanreicherungseinrichtung 4 gefördert wird.

## Patentansprüche

1. Verfahren zur Kultivierung von phototrophen Organismen in wenigstens einem Bioreaktor, bei dem ein wässriges Substrat mindestens einen die phototrophen Organismen aufnehmenden Reaktorraum durchströmt, wobei Nährstoffe aus dem Substrat von den Organismen aufgenommen werden,
**dadurch gekennzeichnet,**
**dass** das Substrat über wenigstens einen mineralischen Festkörper (15) geführt wird, wobei das Substrat an dem wenigstens einen mineralischen Festkörper (15) mit zumindest einem Teil der Nährstoffe angereichert wird und durch den das Substrat hindurchströmt.

2. Verfahren nach Anspruch 1, **dadurch gekennzeichnet, dass** mit dem Substrat Organismen aus dem Reaktorraum (2) ausgeschwemmt werden und die ausgeschwemmten Organismen von dem Substrat abgetrennt werden.

3. Verfahren nach Anspruch 2, **dadurch gekennzeichnet, dass** das Substrat in einem Kreislauf geführt wird, bei dem das Substrat nach dem Abtrennen der ausgeschwemmten Organismen erneut in den Reaktorraum (2) eingeleitet wird.

4. Verfahren nach einem der Ansprüche 1 bis 3, **dadurch gekennzeichnet, dass** das Substrat, bevor es in den Reaktorraum (2) eingeleitet wird, mit natürlichen Mineral- und/oder Nährstoffen angereichert wird.

5. Verfahren nach einem der Ansprüche 1 bis 4, **dadurch gekennzeichnet, dass** zumindest der Reaktorraum (2) temperiert wird.

6. Verfahren nach einem der Ansprüche 1 bis 5, **dadurch gekennzeichnet, dass** der Reaktorraum (2) mit Rauchgas beheizt wird, und das Rauchgas zumindest teilweise in den Reaktorraum (2) eingeleitet wird.

7. Vorrichtung zur Kultivierung von phototrophen Organismen, mit zumindest einem Bioreaktor, der wenigstens einen die phototrophen Organismen aufnehmenden Reaktorraum mit einem zumindest abschnittsweise lichtdurchlässigen Gehäuse aufweist,
**dadurch gekennzeichnet,**
**dass** der Bioreaktor (1) einen durch den Reaktorraum (2) führenden Strömungskanal (5) für ein Substrat zur Nährstoffversorgung der Organismen aufweist, wobei in wenigstens einem Abschnitt des Strömungskanals (5) zumindest ein mineralischer Festkörper (15) angeordnet ist.

8. Vorrichtung nach Anspruch 7, **dadurch gekennzeichnet, dass** dem Reaktorraum (2) wenigstens eine Entnahmeeinrichtung (3) für aus dem Reaktorraum (2) ausgeschwemmte Organismen zugeordnet ist.

9. Vorrichtung nach Anspruch 8, **dadurch gekennzeichnet, dass** zwischen dem Reaktorraum (2) und der Entnahmeeinrichtung (3) ein Substratkreislauf ausgebildet ist.

10. Vorrichtung nach einem der Ansprüche 7 bis 9, **dadurch gekennzeichnet, dass** in einem vom Reaktorraum (2) begrenzten Bereich des Strömungskanals (5) wenigstens abschnittsweise zumindest eine Schüttung aus Granulat angeordnet ist.

11. Vorrichtung nach einem der Ansprüche 7 bis 10, **dadurch gekennzeichnet, dass** in dem Strömungskanal (5) wenigstens eine dem Reaktorraum (2) in Strömungsrichtung des Substrats vorgeschaltete Nährstoffanreichungseinrichtung (4) mit wenigstens einem mineralischen Festkörper (15) angeordnet ist.

12. Vorrichtung nach einem der Ansprüche 7 bis 11, **dadurch gekennzeichnet, dass** innerhalb des Reaktorraums (2) Stege (7, 7') angeordnet sind, die einen mäanderartigen Strömungskanal (5) von einem oberen Ende des Reaktorraums (2) zu einem unteren Ende des Reaktorraums (2) vorgeben.

13. Vorrichtung nach einem der Ansprüche 7 bis 12, **dadurch gekennzeichnet, dass** dem Reaktorraum (2) eine Temperiereinheit (18) zugeordnet ist.

14. Vorrichtung nach einem der Ansprüche 7 bis 13, **dadurch gekennzeichnet, dass** der Reaktorraum (2) wenigstens ein Begasungsventil für einzuleitendes Rauchgas aufweist.

15. Vorrichtung nach einem der Ansprüche 7 bis 14, **dadurch gekennzeichnet, dass** dem Reaktorraum (2) wenigstens ein Begasungsschlauch zugeordnet ist, der zumindest teilweise in dem Reaktorraum (2) angeordnet ist und in dem Reaktorraum zumindest abschnittsweise porös ausgebildet ist.

16. Vorrichtung nach einem der Ansprüche 7 bis 15, **dadurch gekennzeichnet, dass** die Vorrichtung als Fassadenelement oder Dachelement ausgebildet ist.

## Claims

1. A method for cultivating phototrophic organisms in at least one bioreactor, in which an aqueous substrate flows through at least one reactor chamber accommodating the phototrophic organisms, whereupon nutrients are absorbed from the substrate by the organisms, **characterized in that** the substrate is conveyed over at least one mineral solid (15), whereupon the substrate is enriched with at least a portion of the nutrients at the at least one mineral solid (15) through which the substrate flows.

2. The method as claimed in claim 1, **characterized in that** organisms are flushed out of the reactor chamber (2) with the substrate and the flushed organisms are separated from the substrate.

3. The method as claimed in claim 2, **characterized in that** the substrate is conveyed in a circuit whereupon, after separating out the flushed organisms, the substrate is reintroduced into the reactor chamber (2).

4. The method as claimed in one of claims 1 to 3, **characterized in that** before the substrate is introduced into the reactor chamber (2), the substrate is enriched with natural mineral substances and/or nutrients.

5. The method as claimed in one of claims 1 to 4, **characterized in that** at least the reactor chamber (2) is temperature-controlled.

6. The method as claimed in one of claims 1 to 5, **characterized in that** the reactor chamber (2) is heated with flue gas, and at least a portion of the flue gas is introduced into the reactor chamber (2).

7. Apparatus for cultivating phototrophic organisms, having at least one bioreactor which comprises at least one reactor chamber which accommodates the phototrophic organisms and has a housing which is transparent to light at least in sections, **characterized in that** the bioreactor (1) comprises a flow channel (5) leading through the reactor chamber (2) for a substrate to supply the organisms with nutrients, wherein at least one mineral solid (15) is disposed in at least one section of the flow channel (5).

8. The apparatus as claimed in claim 7, **characterized in that** at least one device (3) for extracting organisms flushed out of the reactor chamber (2) is associated with the reactor chamber (2).

9. The apparatus as claimed in claim 8, **characterized in that** a substrate circuit is formed between the reactor chamber (2) and the extraction device (3).

10. The apparatus as claimed in one of claims 7 to 9, **characterized in that** at least one fill formed from granulate is disposed in a zone of the flow channel (5) which is distinct from the reactor chamber (2).

11. The apparatus as claimed in one of claims 7 to 10, **characterized in that** in the flow channel (5) at least one nutrient enrichment device (4) having at least one mineral solid (15) is disposed upstream of the reactor chamber (2) in the direction of flow of the substrate.

12. The apparatus as claimed in one of claims 7 to 11, **characterized in that** fins (7, 7") are provided in the reactor chamber (2) which produce a serpentine type flow channel (5) from an upper end of the reactor chamber (2) to a lower end of the reactor chamber (2).

13. The apparatus as claimed in one of claims 7 to 12, **characterized in that** a temperature control unit (18) is associated with the reactor chamber (2).

14. The apparatus as claimed in one of claims 7 to 13, **characterized in that** the reactor chamber (2) comprises at least one gas supply valve to feed in flue gas.

15. The apparatus as claimed in one of claims 7 to 14, **characterized in that** at least one gas supply pipe is associated with the reactor chamber (2), at least a portion of said gas supply pipe being disposed in the reactor chamber (2) and in the reactor chamber, the gas supply pipe is constructed so as to be porous at least in sections thereof.

16. The apparatus as claimed in one of claims 7 to 15, **characterized in that** the apparatus is constructed as a façade element or roof element.

## Revendications

1. Procédé de mise en culture d'organismes phototrophes dans au moins un bioréacteur, consistant à faire passer un substrat aqueux à travers au moins une chambre de réaction accueillant les organismes phototrophes, les substances nutritives contenues dans ledit substrat étant assimilés par lesdits organismes, **caractérisé en ce que** l'on fait passer ledit substrat sur au moins un corps solide minéral (15) pour ainsi réaliser l'enrichissement dudit substrat en au moins une partie desdites substances nutritives au contact avec l'au moins un corps solide minéral (15) lequel est traversé par ledit substrat.

2. Procédé selon la revendication 1, **caractérisé en ce que** le flux de substrat sortant de la chambre de réaction (2) entraîne des organismes et que les organismes entraînés par ledit flux sont séparés du substrat.

3. Procédé selon la revendication 2, **caractérisé en ce que** ledit substrat est recyclé de manière à ce qu'après la séparation des organismes entraînés par ledit flux, ledit substrat soit de nouveau introduit dans la chambre de réaction (2).

4. Procédé selon l'une des revendications 1 à 3, **caractérisé en ce que** ledit substrat est enrichi en substances minérales et/ou nutritives naturelles avant d'être introduit dans la chambre de réaction (2).

5. Procédé selon l'une des revendications 1 à 4, **caractérisé en ce qu'**au moins la chambre de réaction (2) est thermorégulée.

6. Procédé selon l'une des revendications 1 à 5, **caractérisé en ce que** la chambre de réaction (2) est chauffée au moyen d'un gaz de combustion, ledit gaz de combustion étant au moins partiellement introduit dans la chambre de réaction (2).

7. Dispositif de mise en culture d'organismes phototrophes, comportant au moins un bioréacteur pourvu d'au moins une chambre de réaction accueillant lesdits organismes phototrophe et ayant un boîtier dont au moins certaines parties sont transparentes, **caractérisé en ce que** le bioréacteur (1) comporte un canal d'écoulement (5) qui s'étend à travers la chambre de réaction (2) et qui est destiné à un substrat permettant d'alimenter lesdits organismes en substances nutritives, un corps solide minéral (15) étant disposé dans au moins une partie du canal d'écoulement (5).

8. Dispositif selon la revendication 7, **caractérisé en ce qu'**au moins un dispositif de prélèvement (3), destiné aux organismes ayant quitté la chambre de réaction (2) en étant entraînés par ledit flux, est associé à la chambre de réaction (2).

9. Dispositif selon la revendication 8, **caractérisé en ce qu'**un système de recyclage du substrat est mis en place entre la chambre de réaction (2) et le dispositif de prélèvement (3).

10. Dispositif selon l'une des revendications 7 à 9, **caractérisé en ce qu'**au moins une partie d'un segment une du canal d'écoulement (5), ledit segment étant délimité par la chambre de réaction (2), contient au moins une charge granuleuse disposée en vrac.

11. Dispositif selon l'une des revendications 7 à 10, **caractérisé en ce qu'**au sein du canal d'écoulement (5), au moins un dispositif d'enrichissement en substances nutritives (4) comportant un corps solide minéral (15) est disposé en amont de la chambre de réaction (2) selon le sens d'écoulement dudit substrat.

12. Dispositif selon l'une des revendications 7 à 11, **caractérisé en ce que** des éléments saillants (7, 7') sont disposés au sein de la chambre de réaction (2) pour définir un canal d'écoulement (5) suivant des méandres depuis une extrémité supérieur de la chambre de réaction (2) jusqu'à une extrémité inférieure de la chambre de réaction (2).

13. Dispositif selon l'une des revendications 7 à 12, **caractérisé en ce qu'**une unité de thermorégulation (18) est associée à la chambre de réaction (2).

14. Dispositif selon l'une des revendications 7 à 13, **caractérisé en ce que** la chambre de réaction (2) comporte au moins une soupape d'entrée de gaz permettant d'introduire des gaz de combustion.

15. Dispositif selon l'une des revendications 7 à 14, **caractérisé en ce qu'**au moins un tuyau d'alimentation en gaz est associé à la chambre de réaction (2) et comporte au moins certaines parties qui sont disposées dans la chambre de réaction (2), ledit tuyau comportant, au sein de ladite chambre de réaction, au moins certaines partie qui sont poreuses.

16. Dispositif selon l'une des revendications 7 à 15, **caractérisé en ce que** ledit dispositif est réalisé sous forme d'un élément de façade ou d'un élément de toiture.
